# EUROPEAN PATENT APPLICATION

(11) **EP 0 560 309 A1**
(43) Date of publication of application: **15.09.1993**
(21) Application number: 93103796.4
(22) Date of filing: 10.03.1993
(51) Int. Cl.: A61F 7/02

(54) **Containment and cooling element for application to limbs affected by traumas**

(30) Priority: 11.03.1992 IT MI920565
(71) Applicant: Navarrini, Urano, Milan (IT)
(72) Inventor: Navarrini, Urano, Milan (IT)
(74) Representative: Gervasi, Gemma, Dr.

(57) **Abstract**

The containment and cooling element (70) applicable to limbs affected by traumas which cause swelling comprises a containment enclosure (72) adaptable around the affected limb part. Inside the enclosure in contact with the affected limb part is provided a means which allows circulation of cooling liquid for the limb. The element takes on the form most suitable for the limb part to which it is applied. The cooling liquid is cooled in a preferably portable auxiliary apparatus (94) connected to the containing element (70) by means of two flexible tubes, one for supply and one for return, for circulation of the cooling liquid in the element (70).

## Description

The present invention relates to a containment and cooling element for application to limbs which have undergone distorsions, breaks, sprains, haematomas and more generally traumas causing swelling.

It is known that when a patient suffers a limb trauma of the above type the zone affected undergoes significant swelling due to the discharge of blood caused by breakage of capillaries and muscle fibres and/or sinews if the trauma is in a joint. In this case ice is applied at said zone, which is also protected by a containment wrapping.

If the swelling tends to increase, it is opposed by the wrapping. The latter must therefore be changed to not obstruct swelling of the limb and to avoid serious pain for the patient.

The main purpose of the present invention is to provided a containment element for limbs comprising also cooling means, the containment element replacing the present wrappings and the use of ice.

A second purpose is to provide a containment element of the above type presenting also means of exerting adjustable pressure on the affected limb part.

The above main purpose is achieved by a containment element in accordance with the present invention comprising a containment casing adaptable around the affected limb part. Inside said casing in contact with the affected limb part there being provided a means allowing circulation of a cooling liquid for cooling the affected limb part.

By way of example if the containment element is designed to contain an ankle the containment casing will have approximately the form of a boat shoe while if it is designed for a knee it will have the form of a kneeguard.

To be adaptable to the affected limb part the casing will be opening and closing around the latter. The casing can be for example formed of two parts hinged together to allow slot of the casing after rotation of one part in relation to the other to allow easy introduction of the limb, the two parts being closable around the limb and fixable in position by appropriate closing means.

The containment casing can be formed wholly or partly of substantially rigid material or more or less deformable material depending on the particular requirements of the employment for which it is designed.

If necessary the casing can consist of a folding strip adaptable around the affected limb part and fastenable in position by appropriate fastening means, e.g. strips of VELCRO (registered trademark).

The cooling means for the affected limb part is the type for circulation of an appropriate liquid, the latter being cooled by a conventional refrigerating apparatus.

In accordance to one embodiment of the present invention inside the containment casing an interspace is provided consisting for example of a coil or bladder in which circulates the cooling liquid, said liquid being cooled by separate cooling equipment connected to the containment element by flexible tubes among which is a supply tube and a return tube for the cooling liquid.

Preferably the auxiliary equipment is portable and driven by an electric motor powered by the mains or batteries.

It is convenient that the cooling liquid temperature be thermostat controlled.

The second purpose of the present invention is achieved by providing a substantially rigid casing, at least when closed, and as a jacket in which circulates the cooling liquid an elastic bladder, and a means of controlling the pressure of the cooling liquid circulating in the bladder so that it is possible to vary at will the containment pressure exerted on the affected limb part. In this manner there is secured immobilisation of the part affected with prerogative that said part will be cooled at constant temperature for the time and extension desired (even in 360°).

The invention is clarified by the following description of three embodiments thereof given purely by way of example. In the description reference is made to the annexed drawings wherein:
FIG. 1 is a perspective view of a knee containment element shown open, FIG. 2 is a thigh or calf containment element in folding strip form.
FIG. 3 is a perspective view of an ankle containment element shown closed in which is also visible the above auxiliary apparatus,
FIG. 4 is a longitudinal vertical cross section along plane of cut 4-4 of FIG. 3, and
FIG. 5 is a rough diagram of the apparatus combined with a generic containment element in accordance with the present invention.

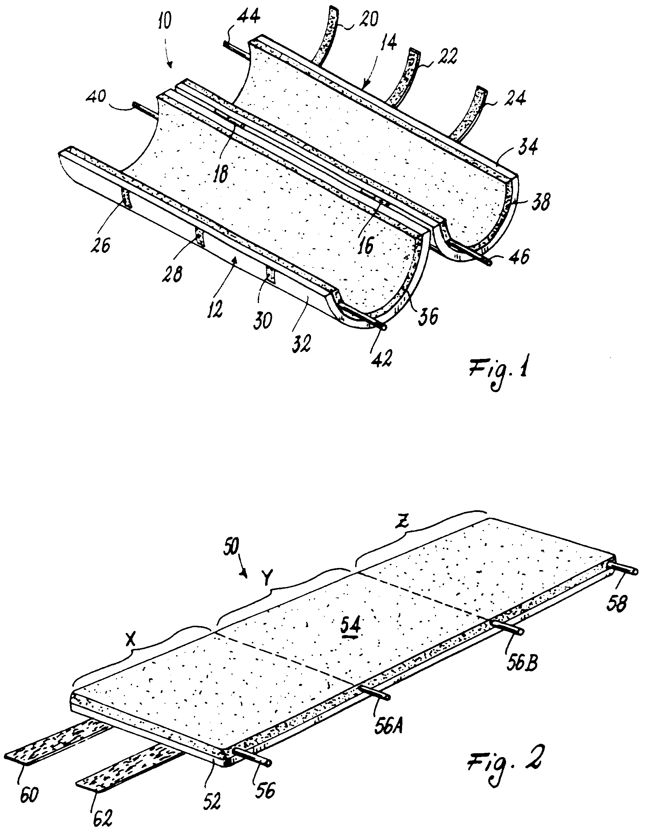

From an examination of FIG. 1 it is seen that the knee containment element 10 consists of two parts: a first substantially rigid part 12 and a second slightly deformable part 14 to better fit the containment element to the patient's knee. The two parts 12 and 14 are connected together by hinges 16 and 18 which permit relative rotation of the two parts 12 and 14. One of the two parts can be superimposed on the other so as to surround the patient's knee requiring containment and cooling. Means of closing and fastening in position of the containment element 10 in this specific case consisting of three strips of VELCRO® 20, 22 and 24 designed to engage with corresponding strips 26, 28 and 30 permit closing and fastening in position of the containment element 10 around the patient's knee in such a manner that the knee is appropriately contained.

Obviously in place of the VELCRO® strips there can be used other means of keeping the containment element 10 closed around the patient's knee.

As seen in FIG. 1 the two parts 12 and 14 consist substantially each of an outer halfcasing or halfshell, 32 and 34, and an elastically deformable soft inner layer 36, 38, e.g. of spongy plastic material permitting better fitting of the containment element 10 around the patient's knee and avoiding excessive local pressure thereon. It is advantageous that the halfshell 32 of the first part 12 o the containment element 10 be substantially rigid while it is appropriate that the halfshell 34 of the second part 14 be slightly deformable to permit better fitting of the element 10 to the patient's knee when the second part 14 is superimposed on the first part 12 and fastened thereto by strips 20,22,24,26,28,30.

In each of said soft paddings 36 and 38 there is inserted a coil (invisible) in which respective ends 40,42 and 44,46 are connected with means of circulating the cooling liquid in the coils, said means being part of an auxiliary apparatus of the type shown in FIG. 5.

In some particular cases it might be necessary to provide cooling only for one of the two parts 12,14 making up the containment element 10. For this purpose it is sufficient to connect with said cooling liquid circulation means only the coil affected while excluding the other.

The coils 40,42 and 44,46 as well as the layers 36,38 of soft material in which they are embedded can be replaced by an elastic bladder arranged inside each of the parts 12 and 14 and in which is circulated the cooling liquid, the bladder functioning also as a soft layer.

As mentioned above the containment device 10 is connected to a preferably portable auxiliary apparatus (not shown in FIG. 1) having the purposes of:
1) circulating the cooling liquid in the cooling jacket (coil or bladder) provided in the containment element;
2) cooling the cooling liquid;
3) controlling the cooling liquid temperature; and optionally, if the jacket walls turned toward the limb are elastically deformable (e.g. an elastic bladder), vary within certain limits the pressure exerted by the cooling liquid on the patient's knee or more generally on the affected limb part or if necessary hold the pressure steady.

An auxiliary apparatus 128 of the above type is represented very schematically in FIG. 5 where reference number 100 indicates a generic containment element in accordance with the present invention and having an inlet 102 and outlet 104 for the cooling liquid.

Said inlet 102 and outlet 104 are connected respectively through corresponding flexible tubes 106 and 108, supply and return respectively, with the outlet 110 and inlet 112 of a heat exchanger 114 in which an evaporator 114A of a cooling circuit 116 cools the cooling liquid, e.g. water, circulating in the containment element 100.

The evaporator 114A is connected through the tubes 118 and 120 to a conventional cooling unit indicated as a whole by reference number 122 comprising a compressor driven by an electric motor, a condenser and a lamination valve (not specifically shown in the figures).

There is also provided a thermostat 124 for controlling the cooling liquid temperature.

A pump 126 permits forced circulation of the cooling liquid. The heat exchanger 114, the cooling unit 122 with associated connection tubes 118 and 120 as well as the pump 126 are part of a single portable unit 128 connected to the containment element through the flexible tubes 106 and 108.

Obviously this does not mean that the auxiliary unit 128 cannot be fixed also and if necessary serve an entire set of containment elements in accordance with the present invention.

In the specific case of FIG. 5 there is provided also an adjustable manostat 210 by means of which it is possible to vary the containment pressure exerted on the affected limb part by the cooling liquid or hold a steady pressure thereon even with change in the swelling of the limb.

FIG. 2 shows a variant 50 of the containment element in accordance with the present invention. Said variant 50 consists essentially of a sturdy folding band which can be adapted around the affected limb part, e.g. the thigh or calf of the patient. In this specific case the containment element 50 comprises a folding casing or external layer 52 made up e.g. of a rather strong fabric which acts as a support and a soft inner layer 54 in which is embedded a flexible coil (not shown). Of the coil is seen only the inlet and outlet ends 56 and 58 respectively to which are connected the associated tubes of the type indicated by reference numbers 106 and 108 of FIG. 5.

The outer casing 52 has closing strips 60 and 62 which are covered with VELCRO® on their upper face.

Once the wrapping containment element 50 is wrapped around e.g. the patient's thigh the strips 60 and 62 are superimposed on corresponding strips (not visible) applied on the outer face of the casing 52 opposite the end of the wrapping element 50 opposite that where there are provided the strips 60 and 62 to fasten in position the containment element 50 on the patient's thigh.

The containment element 50 has the peculiarity of having a cooling zone of variable extent depending on requirements. In particular, in addition to 56, there are provided two other inlets, 56A and 56B respectively for the cooling liquid. Specially provided valves (not visible because located on the lower side of the containment element 50) permit the choice of excluding from circulation the first part of the coil (not visible) which goes from the inlet 56 to the inlet 56A, the cooling liquid being supplied through the latter inlet.

The zone X of the containment element is not cooled in this manner. In a similar manner by specially provided valves (not visible) there can be excluded from circulation both the zones X and Y, supplying the cooling liquid directly through the inlet 56B.

Thanks to the containment device 50 it is thus possible to cool only the part of the limb effectively requiring cooling.

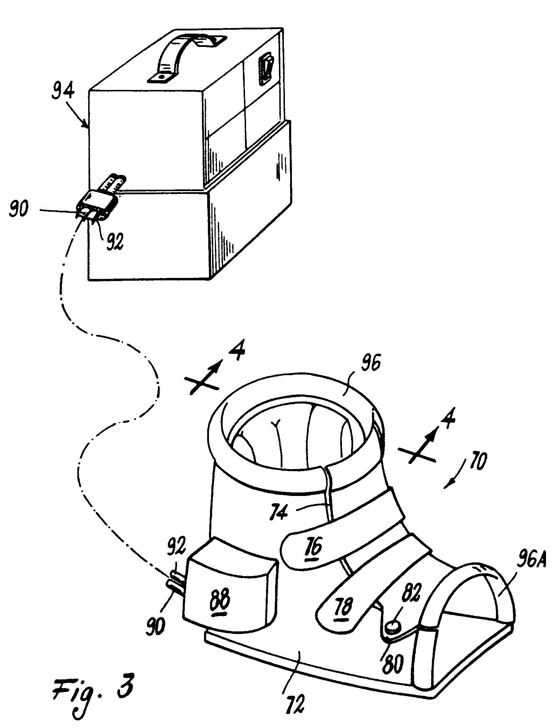

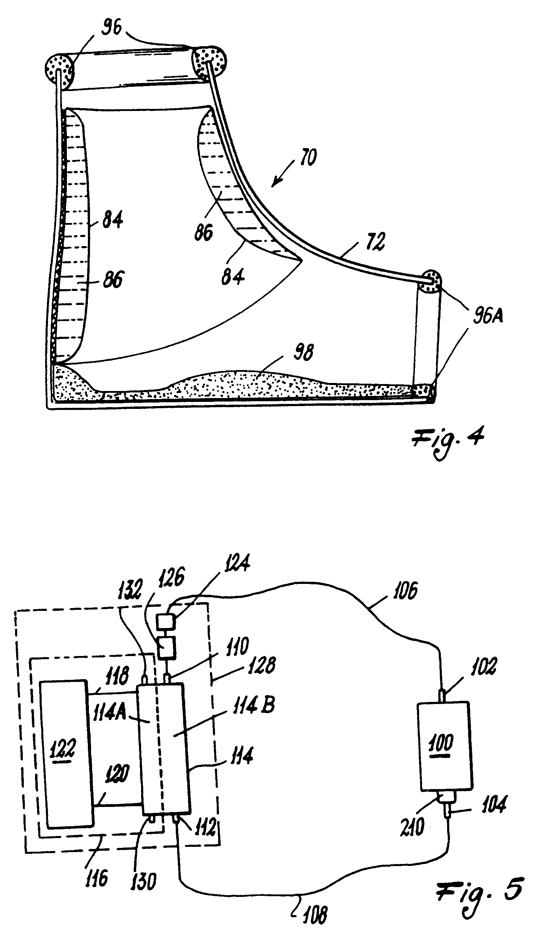

Another variant 70 of the containment element in accordance with the present invention suitable for containing an ankle is visible in FIGS. 3 and 4.

In said FIGS. it is seen how the containment element 70 recalls the form of a boot in which is inserted the patient's foot (not shown). The containment element 70 comprises an outer casing 72 slightly enlargable thanks to the presence of an slot 74. There are also provided means for closing and holding in position the containment element 70 to prevent enlargement of the casing once the foot has been place therein. Said closing means are in this case two closing bands 76 and 78 covered with VELCRO® and fastened to the enclosure 72 and which span the slot 74 to be superimposed on and engaged with corresponding bands (not visible in FIG. 3 because covered by the bands 76 and 78) fastened to the casing 72 from the other side of the slot 74. With the bands 76 and 78 in closed position the casing 72 is substantially rigid.

To increase the rigidity of the casing there is also provided another closing device consisting of a tongue 80 in a single piece with the casing 72 and having a snap fastener 82 engaging in a snapping manner with a corresponding element (not visible) provided on the casing 72 on the part opposite the slot 74.

The latter closing device permits a certain variation (in a V with the vertex at 82) of the amplitude of the slot 74 before closing of the containment element 70 by the bands 76 and 78. This allows better fitting of the casing 72 to the patient's foot.

As may be seen in FIG. 4 the outer casing 72 encloses an elastic bladder 84 in the form of a sleeve surrounding the patient's ankle. In said bladder is circulated water 86 which is fed in through an input and emerges through an outlet (not visible in FIG. 3 because hidden by the box 88 to which lead corresponding flexible tubes 90 and 92 similar to tubes 106 and 108 of FIG. 5) for cold water circulation. Said flexible tubes connect a portable auxiliary unit 94 (analogous to the unit 128 of FIG. 5) to the containment element 70. The unit 94 can be powered by the electricity mains or batteries contained therein for operation in case it cannot be connected to the mains.

In some zones inside the casing 72 not affected by the cooling bladder 84 there is provided a covering of soft material 98 which has the purpose of improving the comfort of the patient who wears the containment element 70. For the same reason a strip of soft material 96 and 96A is also provided opposite the mouth of the upper slot and the mouth of the front slot of the casing 72.

A manostat (not visible in FIG. 3 but indicated by reference number 210 in the diagram of FIG. 5) contained in the box 88 controls the pressure exerted on the affected limb part by the water circulating in the bladder 84. The box 88 has the front wall slot for access to adjustment of the manostat. Therefore, if the ankle swelling reduces, pressure on the limb can be held unchanged by adjusting the manostat.

It is clear that in the embodiments indicated by 10 (FIG. 1) and 50 (FIG. 2) instead of the cooling coils mentioned there can be used as the means allowing circulation of the cooling liquid one or more elastic bladders of the type 84 used in the embodiment 70. As mentioned above, this also allows elimination of the layers of soft material 36, 38 and 54.

## Claims

1. Containment element applicable to limbs, being adaptable around the affected limb part and comprising internal means (40, 42, 44, 46, 56, 58, 84) which allow circulation of cooling liquid.

2. Containment element according to claim 1, having the form of a kneeguard (10) applicable to a knee.

3. Containment element according to claim 2, comprising two parts (12, 14) hinged (16, 18) together to allow slot thereof for introduction of the limb, the two parts (12, 14) being closable around the limb and fastenable in position by closing means (20, 22, 24, 26, 28, 30).

4. Containment element according to claim 3, wherein one (12) of said parts has a substantially rigid outer halfcasing (32) while the other one (14) of said parts has a slightly deformable outer halfcasing (34).

5. Containment element according to claim 4, wherein each of said outer halfcasings (32, 34) bears internally a layer of soft material (36, 38) embedding said internal means (40, 42, 44, 46) in the form of a coil.

6. Containment element according to claim 1, having the form of a folding strip (50) fitting around the affected limb part and fastenable in position by appropriate fastening means (60, 62).

7. Containment element according to claim 6, wherein said folding strip (50) is formed from a strong fabric (52) supporting a soft inner layer (54) embedding said internal means (56, 58) in the form of a flexible coil.

8. Containment element according to claim 7, comprising several inlets (56, 56A, 56B) for the cooling liquid and associated valves for exclusion of some sections of said coil (56, 58) from cooling.

9. Containment element according to claim 1, having the form of a boot (70) applicable to an ankle.

10. Containment element according to claim 9, having a slot (74) allowing introduction of the patient's foot and comprising closing means (76, 78) to prevent enlargement once the foot is introduced.

11. Containment element according to claim 9, comprising an elastic bladder (84) having the form of a sleeve surrounding the patient's ankle and acting as internal means which allow cooling liquid circulation.

12. Containment element according to claim 1, comprising means (110) for controlling the containment pressure exerted on the affected limb part by the cooling liquid circulating in said internal means (84).
